# EUROPÄISCHE PATENTSCHRIFT

(11) **EP 4 146 051 B1**
(45) Veröffentlichungstag und Bekanntmachung des Hinweises auf die Patenterteilung: **18.10.2023**
(21) Anmeldenummer: 21724643.8
(22) Anmeldetag: 07.05.2021
(51) Int. Cl.: A61B 3/02, A61B 3/06

(54) **VORRICHTUNG, VERFAHREN UND COMPUTERPROGRAMM ZUR ERZEUGUNG VON ZWEI PUNKTLICHTQUELLEN DERSELBEN WELLENLÄNGE AUF EINER PUPILLENEBENE EINES AUGES SOWIE ZUR BESTIMMUNG EINER NEURONALEN ÜBERTRAGUNGSFUNKTION EINER SEHBAHN**
DEVICE, METHOD AND COMPUTER PROGRAM FOR PRODUCING TWO POINT LIGHT SOURCES OF THE SAME WAVELENGTH ON A PUPIL PLANE OF AN EYE AND FOR DETERMINING A NEURAL TRANSFER FUNCTION OF A VISUAL PATHWAY
DISPOSITIF, PROCÉDÉ ET PROGRAMME INFORMATIQUE POUR PRODUIRE DEUX SOURCES LUMINEUSES PONCTUELLES DE MÊME LONGUEUR D'ONDE SUR UN PLAN DE PUPILLE D'UN OEIL ET POUR DÉTERMINER UNE FONCTION DE TRANSFERT NEURONAL D'UN TRAJET VISUEL

(30) Priorität: 08.05.2020 DE 102020205851
(43) Veröffentlichungstag der Anmeldung: 15.03.2023
(73) Patentinhaber: Carl Zeiss Vision International GmbH, 73430 Aalen (DE)
(72) Erfinder: LEUBE, Alexander, 73434 Aalen (DE); SUCHKOV, Nikolai, 72127 Kusterdingen (DE); SCHWARZ, Christina, 72127 Kusterdingen (DE); WAHL, Siegfried, 73072 Donzdorf (DE)
(74) Vertreter: Altmann Stößel Dick Patentanwälte PartG mbB
(86) Internationale Anmeldenummer: PCT/EP2021/062126
(87) Internationale Veröffentlichungsnummer: WO 2021/224454

(56) Entgegenhaltungen:
- CN-A- 102 335 088
- US-A- 5 396 303
- US-A- 5 579 161
- SARA CASTILLO AND BRIAN VOHNSEN: "Exploring the Stiles-Crawford effect of the first kind with coherent light and dual Maxwellian sources", APPLIED OPTICS, OPTICAL SOCIETY OF AMERICA, WASHINGTON, DC, US, Bd. 52, Nr. 1, 1. Januar 2013 (2013-01-01) , Seiten A1-A8, XP001580202, ISSN: 0003-6935, DOI: HTTP://DX.DOI.ORG/10.1364/AO.52.0000A1
- SEKIGUCHI N ET AL: "ABERRATION-FREE MEASUREMENTS OF THE VISIBILITY OF ISOLUMINANT GRATINGS", JOURNAL OF THE OPTICAL SOCIETY OF AMERICA A,, Bd. 10, Nr. 10, 1. Oktober 1993 (1993-10-01), Seiten 2105-2117, XP000198126, ISSN: 1084-7529
- WILLIAMS ET AL: "Aliasing in human foveal vision", VISION RESEARCH, ELSEVIER, AMSTERDAM, NL, Bd. 25, Nr. 2, 1. Januar 1985 (1985-01-01) , Seiten 195-205, XP024311209, ISSN: 0042-6989, DOI: 10.1016/0042-6989(85)90113-0 [gefunden am 1985-01-01]
- KATSUHIKO KOBAYASHI ET AL: "Calculation of ocular single-pass modulation transfer function and retinal image simulation from measurements of the polarized double-pass ocular point spread function", JOURNAL OF BIOMEDICAL OPTICS, Bd. 9, Nr. 1, 1. Januar 2004 (2004-01-01), Seite 154, XP055108985, ISSN: 1083-3668, DOI: 10.1117/1.1627777

## Beschreibung

### Gebiet der Erfindung

Die vorliegende Erfindung betrifft eine Vorrichtung, ein Verfahren und ein Computerprogramm zur Erzeugung von zwei Punktlichtquellen derselben Wellenlänge auf einer Pupillenebene mindestens eines Auges eines Nutzers sowie eine diese umfassende Vorrichtung und ein damit in Beziehung stehendes Verfahren und Computerprogramm zur Bestimmung einer neuronalen Übertragungsfunktion einer Sehbahn des Nutzers.

### Stand der Technik

Das menschliche Sehen ist ein komplexer Vorgang, der in einen optischen Beitrag, eine Modulationsübertragungsfunktion und eine neuronale Übertragungsfunktion gegliedert werden kann. Die neuronale Übertragungsfunktion einer Sehbahn eines Nutzers beschreibt hierbei einen neuronalen Beitrag zum menschlichen Sehen. Der Begriff der "Sehbahn" *(engl.* visual pathway) bezeichnet dabei eine Kette von Organen, die dazu eingerichtet ist, einem Nutzer das Sehen zu ermöglichen, wobei die Kette der Organe mindestens ein Auge, eine Retina, einen Sehnerv sowie alle Zentren im Gehirn, die zum Sehen beitragen, umfasst. Die "Sehbahn" wird auch "visuelles System" genannt. Die neuronale Übertragungsfunktion stellt somit einen Hauptfaktor für eine verhaltensbedingte Kontrastwahrnehmung beim Sehen dar. Eine Kenntnis der neuronalen Übertragungsfunktion ermöglicht es, optische Behandlungen mittels Brille, Brillengläsern, Kontaktlinsen, Intraokularlinsen und/oder refraktiver Chirurgie auf einen ausgewählten Nutzer anzupassen. Weiterhin kann die neuronale Übertragungsfunktion als diagnostisches Werkzeug für neuronale Störungen und zur Schulung von Wahrnehmung verwendet werden.

Aus dem Stand der Technik sind Vorrichtungen und Verfahren zur Bestimmung der neuronalen Übertragungsfunktion der Sehbahn eines Nutzers bekannt, die auf einem analytischen Verfahren oder auf einem interferometrischen Verfahren basieren. Die analytischen Verfahren umfassen eine separate Messung des Liquors und der Modulationsübertragungsfunktion, woraufhin die neuronale Übertragungsfunktion analytisch mittels mathematischer Modelle bestimmt wird. Die analytischen Verfahren erwiesen sich jedoch als zeitaufwändig und ungenau.

DE 10 2008 041 458 B4 offenbart ein Verfahren zur Bestimmung der neuronalen Kontrastempfindlichkeitsfunktion eines Auges mit folgenden Verfahrensschritten:
a) Bestimmung der optischen Kontrastempfindlichkeitsfunktion des Auges;
b) Bestimmung der physiologischen Kontrastempfindlichkeitsfunktion des Auges;
c) Berechnung der neuronalen Kontrastempfindlichkeitsfunktion des Auges durch Division aus den zuvor bestimmten optischen und physiologischen Kontrastempfindlichkeitsfunktionen des Auges, wobei die optische Kontrastempfindlichkeitsfunktion des Auges aus einer Mittelung aus mehreren zuvor bestimmten optischen Kontrastempfindlichkeitsfunktionen und/oder wobei die physiologische Kontrastempfindlichkeitsfunktion (CSF) des Auges aus einer Mittelung aus mehreren zuvor bestimmten physiologischen Kontrastempfindlichkeitsfunktionen und/oder wobei die neuronale Kontrastempfindlichkeitsfunktion des Auges aus einer Mittelung aus mehreren zuvor berechneten neuronalen Kontrastempfindlichkeitsfunktionen des Auges berechnet wird.

M. Mon-Williams, J.R. Tresilian, N.C. Strang, P. Kochhar und J.P. Wann, Improving vision: neural compensation for optical defocus, Proc. R. Soc. Lond. B 265, 1998, S. 71-77, beschreiben eine deutliche Verbesserung der Fähigkeit, Buchstaben zu erkennen, nachdem die Augen eines Nutzers längere Zeit keiner refraktiven Korrektur ausgesetzt waren. Hierzu wurde in einem ersten Experiment an jedem Probanden zunächst die optische Kontrastempfindlichkeitsfunktion des Auges gemessen. Dann wurde in einem zweiten Experiment die physiologische Kontrastempfindlichkeitsfunktion des Auges erfasst und hieran anschließend eine umfangreiche Auswertung vorgenommen.

In den interferometrischen Verfahren werden zwei kohärente Lichtstrahlen verwendet, die in das Auge des Nutzers projiziert werden und in der Retina des Auges interferieren. Hierzu erfolgt üblicherweise eine mechanische Steuerung des Lichtstrahls, die die Geschwindigkeit und Effizienz einer zugehörigen Vorrichtung begrenzt, die aufgrund eines kompliziert eingerichteten Strahlengangs eine verringerte Stabilität und Robustheit aufweist. Derartige Vorrichtungen lassen sich insbesondere aufgrund ihrer Anfälligkeit für Vibrationen weder in einer klinischen Umgebung noch in einem kommerziellen Produkt einsetzen. Darüber hinaus erfordert eine Einstellung von Kontrasten in den Interferenzstreifen ein optisches Hilfselement, wodurch der Aufbau der Vorrichtung noch komplexer wird. Die bekannten Vorrichtungen können daher nur von einem Fachmann auf dem Gebiet der Strahlenoptik bedient werden.

D.R. Williams, Aliasing in Human Foveal Vision, Vision Res. 25(2), 1985, S. 195-205, beschreibt ein Laserinterferometer, das Kontrastempfindlichkeitsmessungen ermöglicht, die von optischen Unschärfen im Auge weitgehend unbeeinflusst sind. Ein Strahlteiler teilt Licht aus einem Laser in zwei spiegelbildlich zueinander angeordnete Lichtstrahlen auf. Jeder Lichtstrahl beaufschlagt einen akusto-optischen Modulator, mit dem sich Kontrast und räumliche Phase der Interferenzstreifen einstellen lassen. Ein in den jeweiligen Strahlengang eingebrachtes räumliches Filter, das ein Mikroskop-Objektiv und eine Apertur-Blende aufweist, entfernt räumliches Rauschen aus jedem Lichtstrahl und weitet diesen jeweils auf. Eine jeweilige Kombination aus zwei optischen Linsen und einem Spiegel kollimiert den jeweils aufgeweiteten Lichtstrahl und erzeugt jeweils ein Abbild der betreffenden Apertur-Blende innerhalb eines Glaswürfels. Die beiden Lichtstrahlen kreuzen sich in dem Glaswürfel, wobei sie sich in fast entgegengesetzte Richtungen ausbreiten, wodurch sich eine Raumfrequenz der Interferenzstreifen einstellen lässt. Eine weitere Kombination aus jeweils einem Spiegel und einer kollimierenden Linse führt die beiden Lichtstrahlen zurück zum Strahlteiler, wo sie rekombinieren und anschließend eine Feldblende beaufschlagen, welche die Interferenzstreifen auf der Retina des Auges abbildet. Durch Einstellen eines Abstandes der Feldblende von einer weiteren Linse lässt sich die Feldblende fokussieren; alternativ kann dazu ein weiterer Strahlteiler verwendet werden. Unmittelbar vor dem Auge befindet sich ein linearer Polarisator, der dazu eingerichtet ist, depolarisiertes Streulicht zu entfernen, um eine Verringerung des Kontrasts der Interferenzringe zu vermeiden. Zur Verminderung von Vibrationen werden ein vibrationsgedämpfter optischer Tisch, gefaltete Strahlengänge und eine feste Kopplung der Spiegel und Strahlteiler vorgeschlagen.

N. Sekiguchi, D.R. Williams und D.H. Brainardt, Aberration-free measurements of the visibility of isoluminant gratings, J. Opt. Soc. Am. A 10 (10), 1993, S. 2105-2117, offenbaren die Merkmale des Oberbegriffs des unabhängigen Anspruchs 1 und beschreiben eine Vorrichtung, die zwei identische Laserinterferometer umfasst, wobei jeder der beiden Laserinterferometer dazu eingerichtet ist, Interferenzstreifen auf der Retina des Auges des Nutzers zu erzeugen, wobei sich eine relative Phase zwischen den von den beiden Laserinterferometern erzeugten Interferenzstreifen einstellen lässt. Auch hierbei umfasst jedes Laserinterferometer zwei akusto-optische Modulatoren, mit dem sich der Kontrast und die räumliche Phase der Interferenzringe einstellen lassen, ein räumliches Filter, das ein Mikroskop-Objektiv und eine Apertur-Blende aufweist, und eine Feldblende, welche die Interferenzstreifen auf der Retina des Auges abbildet.

US 2008/0309873 A1 offenbart ein Verfahren zur Korrektur von Aberrationen des Auges, angewandt auf ein ophthalmologisches Instrument, das mit einem Analyselichtstrahl arbeitet, umfassend: Messung von Aberrationen des Auges, die mit dem Analysestrahl interferieren, Korrektur der Phase der Wellenfront des Analysestrahls als Funktion der gemessenen Werte der Aberrationen, Messung von Augenbewegungen unabhängig von der Messung der Aberrationen, und Modifikation der Korrektur der Phase der Wellenfront des Analysestrahls als Funktion der Messung der Augenbewegungen.

In Williams, David R., et al., Double pass and interferometric measures of the optical quality of the eye, JOSA A, 1994, 11. Jg., Nr. 12, S. 3123-3135, werden zwei Verfahren zur Messung der Modulationsübertragungsfunktion (MTF) des menschlichen Auges verglichen, ein interferometriesches Verfahren und ein Double-Pass-Verfahren. Für beide Verfahren wurden derselbe Beobachter, derselbe Brechungszustand, dieselbe Pupillengröße (3 mm) und dieselbe Wellenlänge (632,8 nm) verwendet. Bei dem Doppelpass-Verfahren wurde eine enge Übereinstimmung zwischen der Ebene des subjektiv besten Fokus für den Beobachter und der Ebene des objektiv besten Fokus gefunden, was darauf hindeutet, dass ein Großteil des reflektierten Lichts während des doppelten Durchgangs durch die Rezeptorschicht auf die einzelnen Zapfen beschränkt ist. Im Doppelpass-Verfahren erzeugte MTFs wiesen einen Verlust in der Modulationsübertragung auf, der wahrscheinlich auf das von der Aderhaut reflektierte Licht zurückzuführen ist.

Sara Castillo und Brian Vohnsen, Exploring the Stiles-Crawford effect of the first kind with coherent light and dual Maxwellian sources, Applied Optics, 2013, Vol. 52, No. 1 offenbaren die Merkmale des Oberbegriffs des unabhängigen Anspruchs 1 und beschreiben, dass die visuelle Wirkung von Licht, das schräg auf die Netzhaut trifft, durch den Stiles-Crawford-Effekt der ersten Art vermindert wird. Normalerweise wird er analysiert, indem eine kleine Maxwell'sche Quelle über die Pupille des Auges gescannt wird, während subjektive Sichtbarkeitsvergleiche mit einem statischen Referenzfeld angestellt werden, das in der Nähe der Pupillenmitte in das Auge eintritt. In der Veröffentlichung wird eine alternative Charakterisierungsmethode mit zwei kohärenten Maxwell` schen Punktquellen vorgeschlagen, die sich auf gegenüberliegenden Seiten der Pupille befinden. Dies führt zu Interferenzstreifen auf der Netzhaut mit einem zugrundeliegenden Phasengradienten. Durch Änderung des Leistungsverhältnisses der beiden Punktquellen kann die Wellenfrontneigung auf der Netzhaut eingestellt werden. So kann der Stiles-Crawford-Effekt der ersten Art untersucht werden, ohne dass das einfallende Licht über die Pupille gescannt wird. Hierzu wurde ein räumlicher Lichtmodulator mit holografischen Phasenkarten verwendet, um zwei Maxwell` sche Punktquellen an der Pupille zu erzeugen, die eine bestimmte Phasenvariation auf die Retina projizieren.

### Aufgabe der Erfindung

Insbesondere vor dem Hintergrund von N. Sekiguchi, s.o., besteht die Aufgabe der vorliegenden Erfindung darin, eine Vorrichtung, ein Verfahren und ein Computerprogramm zur Erzeugung von zwei Punktlichtquellen derselben Wellenlänge auf einer Pupillenebene mindestens eines Auges eines Nutzers sowie eine Vorrichtung, ein Verfahren und ein Computerprogramm zur Bestimmung einer neuronalen Übertragungsfunktion einer Sehbahn des Nutzers bereitzustellen, welche die aufgeführten Nachteile und Einschränkungen des Standes der Technik zumindest teilweise überwinden.

Insbesondere sollen die Vorrichtungen kompakt und robust aufgebaut sein und so eine einfache Bedienung ermöglichen, um in einem kommerziellen Produkt in einer klinischen Umgebung eingesetzt werden zu können.

### Offenbarung der Erfindung

Diese Aufgabe wird gelöst durch eine Vorrichtung, ein Verfahren und ein Computerprogramm zur Erzeugung von mindestens zwei Punktlichtquellen derselben Wellenlänge auf einer bzw. der jeweiligen Pupillenebene mindestens eines Auges eines Nutzers sowie durch eine Vorrichtung, ein Verfahren und ein Computerprogramm zur Bestimmung einer neuronalen Übertragungsfunktion einer Sehbahn des Nutzers mit den Merkmalen der unabhängigen Patentansprüche. Bevorzugte Ausgestaltungen, welche einzeln oder in Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben dem durch diese Begriffe eingeführten Merkmal, keine weiteren Merkmale vorhanden sind oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist, d.h. auf eine Situation, in welcher A ausschließlich aus B besteht, als auch auf die Situation, in welcher, zusätzlich zu B, ein oder mehrere weitere Elemente in A vorhanden sind, beispielsweise Element C, Elemente C und D oder sogar weitere Elemente.

In einem ersten Aspekt betrifft die vorliegende Erfindung eine Vorrichtung zur Erzeugung von mindestens zwei Punktlichtquellen derselben Wellenlänge auf einer bzw. der jeweiligen Pupillenebene mindestens eines Auges eines Nutzers. Wie allgemein bekannt, bezeichnet der Begriff der "Punktlichtquelle" eine Lichtquelle, die eine vernachlässigbare Ausdehnung in Bezug auf ihre Entfernung zu dem beleuchteten Objekt aufweist. Im Falle der vorliegenden Erfindung werden mindestens die beiden Punktlichtquellen derselben Wellenlänge auf einer bzw. der jeweiligen Pupillenebene mindestens eines Auges eines Nutzers erzeugt. Anstelle des hier verwendeten Begriffs des "Nutzers" kann gleichbedeutend auch einer der Begriffe "Subjekt", "Brillenträger", "Benutzer" oder "Proband" verwendet werden. Der Begriff "Pupillenebene" betrifft hierbei eine durch die als "Pupille" bezeichnete Eintrittsöffnung in den Augapfel legbare und durch einen Umfang der Pupille begrenzte Ebene. Weiterhin bezeichnet die "Retina" im Rahmen der vorliegenden Erfindung eine sich am Hintergrund des Augapfels befindliche auch als "Netzhaut" bezeichnete photosensitive Schicht, die typischerweise von einem Rand der Pupille bis zu einer Austrittsstelle des Sehnervs ausgedehnt ist.

Die Vorrichtung zur Erzeugung von mindestens zwei Punktlichtquellen derselben Wellenlänge auf einer bzw. der jeweiligen Pupillenebene mindestens eines Auges eines Nutzers umfasst:
- mindestens eine kohärente Lichtquelle zur Erzeugung mindestens eines Lichtstrahls;
- mindestens eine optische Einrichtung zur Aufteilung des mindestens einen Lichtstrahls jeweils in zwei Paare von Teillichtstrahlen, zur Überlagerung der jeweiligen Teillichtstrahlen in jedem Paar und zur Einstellung von Kontrast und räumlicher Phase in mindestens einem aus der Überlagerung der beiden Teillichtstrahlen für jedes Paar gebildeten Interferenzmuster; und
- mindestens einen Strahlengang zur Führung jedes Paares der überlagerten Teillichtstrahlen derart, dass mindestens zwei Punktlichtquellen derselben Wellenlänge auf einer bzw. der jeweiligen Pupillenebene mindestens eines Auges eines Nutzers erzeugt werden;
wobei die mindestens eine optische Einrichtung mindestens ein digitales Lichtmodulationselement aufweist, wobei das digitale Lichtmodulationselement eine optische Vorrichtung ist, die eine Vielzahl an einzeln ansteuerbaren optischen Elementen aufweist, die zur Modulation des eintreffenden Lichtstrahls eingerichtet sind; und wobei die mindestens eine kohärente Lichtquelle mindestens eine polychromatische Lichtquelle mit einer Superkontinuum-Laserquelle und mindestens ein abstimmbares Wellenlängenfilter umfasst.

Die vorliegende Vorrichtung zur Erzeugung von mindestens zwei Punktlichtquellen derselben Wellenlänge auf einer bzw. der jeweiligen Pupillenebene mindestens eines Auges eines Nutzers umfasst mindestens eine kohärente Lichtquelle, die zur Erzeugung mindestens eines Lichtstrahls eingerichtet ist. Der Begriff der "kohärenten Lichtquelle" bezeichnet eine Lichtquelle, die zur Erzeugung von kohärentem Licht eingerichtet ist, das sich in Form von Lichtstrahlen ausbreitet, die innerhalb der Vorrichtung entlang eines festgelegten Strahlengangs geführt werden. Hierbei weisen die von der Lichtquelle emittierten Lichtstrahlen untereinander eine zeitlich stabile Phasenbeziehung auf, wodurch eine zeitlich stabile Interferenz zwischen den Lichtstrahlen oder hiervon abgezweigten Teillichtstrahlen ermöglicht wird. Der Begriff der "Interferenz" bezieht sich hierbei auf eine Überlagerung zweier Lichtstrahlen oder Teillichtstrahlen, die bei kohärentem Licht ein zeitlich stabiles Interferenzmuster, insbesondere in Form von parallel zueinander angeordneten Interferenzstreifen in einer oder in zwei Dimensionen, die vorzugsweise eine feste Periode aufweisen, ausbilden. Ein derartiges zweidimensionales Muster kann ausgewählt sein aus einem Schachbrettmuster; und/oder aus alternierenden Strukturen, die insbesondere Ringe oder Linien umfassen können; und/oder aus attenuierenden Strukturen.

Als die mindestens eine kohärente Lichtquelle hierfür eignet sich erfindungsgemäß mindestens eine polychromatische Lichtquelle mit mindestens einer Superkontinuum-Laserquelle. Laserlicht wird als "Superkontinuum" bezeichnet, wenn es nach Durchquerung eines nichtlinearen optischen Mediums ein breitbandiges optisches Spektrum aufweist, das einen Frequenzbereich von mindestens einer Oktave umfassen kann. Die Verwendung mindestens einer Superkontinuum-Laserquelle ermöglicht es, wie unten näher ausgeführt, in vorteilhafter Weise eine Bereitstellung von kohärentem Laserlicht mehrerer Wellenlängen, wobei die erfindungsgemäße Vorrichtung zusätzlich mindestens ein abstimmbares Wellenlängenfilter umfasst, das zur Auswahl eines schmalbandigen Bereichs aus dem breitbandigen Bereich eingerichtet ist. Hierbei ermöglicht die Kombination aus der mindestens einen Superkontinuum-Laserquelle und dem mindestens einen abstimmbaren Wellenlängenfilter eine schnelle Bestimmung der neuronalen Übertragungsfunktion über das gesamte sichtbare Spektrum oder einen Teil davon.

Die vorliegende Vorrichtung zur Erzeugung von mindestens zwei Punktlichtquellen derselben Wellenlänge auf einer bzw. der jeweiligen Pupillenebene mindestens eines Auges eines Nutzers umfasst weiterhin eine optische Einrichtung, die
- zur Aufteilung des Lichtstrahls in mindestens zwei Paare von Teillichtstrahlen;
- zur Überlagerung der jeweiligen Teillichtstrahlen in jedem Paar; und
- zur Einstellung von Kontrast und räumlicher Phase in einem aus der Überlagerung der beiden Teillichtstrahlen für jedes Paar gebildeten Interferenzmuster
eingerichtet ist. Hierbei bezeichnet der Begriff des "Teillichtstrahls" einen Lichtstrahl, den ein eintreffender Lichtstrahl in Folge einer Durchquerung eines hierfür eingerichteten optischen Elements erzeugt, während der Begriff "Aufteilung" eine Aufspaltung des eintreffenden Lichtstrahls in mindestens zwei Teillichtstrahlen betrifft. Im Falle der vorliegenden Erfindung wird mindestens ein eintreffender Lichtstrahl derart aufgeteilt, dass mindestens zwei Paare von Teillichtstrahlen gebildet werden, wobei jedes Paar zwei einzelne Teillichtstrahlen umfasst, die, nach Durchquerung eines Teilstücks des Strahlengangs, wieder miteinander überlagert werden. Aufgrund der kohärenten Natur der einzelnen Teillichtstrahlen bildet sich, wie oben und unten näher erläutert, aus der Überlagerung der beiden Teillichtstrahlen in jedem Paar ein Interferenzmuster aus, das insbesondere in Form von parallel zueinander angeordneten Interferenzstreifen oder als Schachbrettmuster vorliegen kann. Der Begriff der "Überlagerung" bezieht sich hierbei auf eine Führung der beiden Teillichtstrahlen in jedem Paar in einer Weise, dass die getrennt geführten Teillichtstrahlen schließlich am selben Ort im Strahlengang aufeinandertreffen, um so eine Superposition ihrer Amplituden zu bilden. Hierbei ist die optische Einrichtung derart eingerichtet, dass Kontrast und räumliche Phase in für jedes Paar gebildeten Interferenzmustern einstellbar sind.

Während aus dem Stand der Technik bekannte derartige optische Einrichtungen eine Mehrzahl an optischen Komponenten, insbesondere mehrere Strahlteiler, akusto-optische Modulatoren, Glaswürfel, Spiegel und Linsen, die in genau zueinander ausgerichteten Positionen angeordnet sind, umfassen, wird erfindungsgemäß vorgeschlagen, mindestens ein digitales Lichtmodulationselement als optische Einrichtung zu verwenden. Der Begriff des "digitalen Lichtmodulationselements" bezeichnet hierbei eine optische Vorrichtung, die eine Vielzahl an einzeln ansteuerbaren optischen Elementen aufweist, die zur Modulation eines eintreffenden Lichtstrahls eingerichtet sind.

Bevorzugt kann das mindestens eine digitale Lichtmodulationselement mindestens einen räumlichen Lichtmodulator oder mindestens eine digitale Mikrospiegel-Einheit aufweisen. Der Begriff des "räumlichen Lichtmodulators" (engl. "Spatial Light Modulator" oder kurz "SLM") bezeichnet eine optische Einrichtung, die zur Aufprägung eines Intensitätsmusters, insbesondere in Form einer räumlichen Modulation und/oder einer Phase, auf einen eintreffenden Lichtstrahl eingerichtet ist, wobei die Aufprägung des Intensitätsmusters elektronisch und/oder optisch erfolgen kann. Aus diesem Grund eignet sich der räumliche Lichtmodulator üblicherweise zur Pulsformung und/oder zur Erzeugung von optischen Gittern. Die digitale Mikrospiegel-Einheit (engl. "Digital Micromirror Device", kurz "DMD") wird in der Regel dazu verwendet, ein digitales Bild auf einen Lichtstrahl zu modulieren. Hierzu weist die digitale Mikrospiegel-Einheit eine Anordnung umfassend eine Vielzahl an matrixförmig angeordneten verkippbaren Mikrospiegeln auf, die über eine Kantenlänge im Mikrometerbereich verfügen, wobei jeder Mikrospiegel mittels elektrostatischen Feldern einzeln einstellbar ist. Ein eintreffender Lichtstrahl wird durch diese Anordnung in einzelne Pixel zerlegt und dann pixelweise reflektiert. Andere Arten von digitalen Lichtmodulationselementen sind jedoch möglich.

Im Rahmen der hier vorliegenden Erfindung kann das mindestens eine digitale Lichtmodulationselement vorzugsweise dazu eingerichtet sein, den Kontrast und die räumliche Phase in einem aus der Überlagerung der beiden Teillichtstrahlen für jedes Paar gebildeten Interferenzmuster einzustellen. Insbesondere lässt sich, wie unten erwähnt, durch eine Festlegung eines Verhältnisses zwischen den Flächen von zwei Pupillen, was dem jeweiligen Paar der Teillichtstrahlen zugeordneten Ringen auf dem mindestens einen digitalen Lichtmodulationselement entspricht, der jeweilige Kontrast des Interferenzmusters, insbesondere der Interferenzstreifen oder des zweidimensionalen Musters, einstellen. Vorzugsweise kann hierzu das mindestens eine digitale Lichtmodulationselement dazu eingerichtet sein, eine isoluminante Einstellung des Kontrasts in dem für jedes Paar gebildeten Interferenzmuster vorzunehmen, indem die Flächen der beiden, dem jeweiligen Paar der Teillichtstrahlen zugeordneten Ringen auf dem mindestens einen digitalen Lichtmodulationselement gleichgroß gewählt werden. Alternativ oder zusätzlich kann die Einstellung des Kontrasts in dem für jedes Paar gebildeten Interferenzmuster mittels einer Änderung einer Modulationstiefe erfolgen, wobei bevorzugt für eine Pupille eine Beugungseffizienz für die erste Ordnung verringert werden kann. Ein derartiges Vorgehen ermöglicht eine Vornahme von Kontraständerungen in den jeweiligen Interferenzmustern mit einer Wiederholrate des mindestens einen digitalen Lichtmodulationselements, z.B. mit 10 Hz bis 100 Hz, bevorzugt mit 25 Hz bis 50 Hz, insbesondere mit 30 Hz, während zugleich eine Leuchtdichte der beiden Teillichtstahlen konstant gehalten werden kann. Der Begriff der "Leuchtdichte" bezeichnet eine Flächenhelligkeit, mit welcher das mindestens eine Auge eines Nutzers eine beleuchtete Fläche wahrnehmen kann.

Im Rahmen der hier vorliegenden Erfindung kann das mindestens eine digitale Lichtmodulationselement vorzugsweise dazu eingerichtet sein, eine laterale Verschiebung der Teillichtstrahlen eines der Paare der Teillichtstrahlen in Bezug auf eine Ausbreitungsrichtung der Teillichtstrahlen eines der anderen der Paare der Teillichtstrahlen vorzunehmen. Auf diese Weise kann das mindestens eine digitale Lichtmodulationselement einen eintreffenden kohärenten Lichtstrahl in zwei symmetrisch zueinander geneigte Paare von Teillichtstrahlen aufteilen. Wie unten näher erläutert, lässt sich hierbei mindestens ein Parameter des Interferenzmusters, insbesondere Richtung und Raumfrequenz der Interferenzstreifen in der einen oder in den beiden Dimensionen, durch Einstellung der Richtung und Steigung einer Neigung der Teillichtstrahlen des ausgewählten Paares der Teillichtstrahlen festlegen.

Mittels eines dafür eingerichteten Strahlengangs zur Führung jedes Paares der überlagerten Teillichtstrahlen werden schließlich mindestens die beiden gewünschten Punktlichtquellen derselben Wellenlänge auf der jeweiligen Pupillenebene des mindestens einen Auges des Nutzers erzeugt. Hierbei kann der Strahlengang zur Führung jedes Paares der überlagerten Teillichtstrahlen bevorzugt derart eingerichtet sein, dass mindestens die beiden Interferenzmuster jeweils auf der Retina, d.h. auf der lichtempfindlichen Schicht, des mindestens einen Auges des Nutzers abgebildet werden.

In einer bevorzugten Ausgestaltung der vorliegenden Vorrichtung kann zusätzlich zur Neigung der Teillichtstrahlen mindestens eines Paares eine gemeinsame Neigung auf mindestens beide Paare der Teillichtstrahlen angewandt werden, insbesondere um moduliertes Licht von nicht-moduliertem Licht, das jeweils aus dem mindestens einen digitalen Lichtmodulationselement geführt wird, zu trennen. Hierzu kann eine optische Achse des mindestens einen digitalen Lichtmodulationselements vorzugsweise derart ausgerichtet sein, dass nicht-moduliertes Licht mittels mindestens eines Filters, das zur Entfernung von nicht-moduliertem Licht aus dem Lichtstrahl nach dem Durchqueren des mindestens einen digitalen Lichtmodulationselements eingerichtet ist, aus dem Lichtstrahl entfernt werden kann. Zu diesem Zweck kann das mindestens eine Filter bevorzugt mindestens eine Apertur-Blende aufweisen oder umfassen. Der Begriff der "Apertur-Blende" bezeichnet hierbei ein optisches Element, das eine, vorzugsweise verstellbare, Öffnung aufweist, durch die ein Lichtstrahl oder ein Teil davon eintreten und so die Blende durchqueren kann.

In einer weiteren bevorzugten Ausgestaltung der vorliegenden Vorrichtung kann mindestens ein strahlaufweitendes, kollimierendes optisches Element zwischen der mindestens einen kohärenten Lichtquelle und dem mindestens einen digitalen Lichtmodulationselement angeordnet sein. Das mindestens eine strahlaufweitende, kollimierende optische Element kann hierbei bevorzugt mindestens einen Diffusor, mindestens eine optische Linse oder mindestens einen Strahlteiler umfassen. Als "Diffusor" wird hierbei ein optisches Element bezeichnet, das dazu eingerichtet ist, Licht zu streuen, insbesondere mittels diffuser Reflexion und/oder Lichtbrechung. Ein Einsatz des mindestens einen strahl aufweitenden, kollimierenden optischen Elements kann es insbesondere ermöglichen, eine Sichtbarkeit von Specklemustern, die durch das kohärente Licht erzeugt werden, zu verringern. Dadurch kann insbesondere eine Messgenauigkeit bei geringen Kontrasten erhöht werden.

Grundsätzlich kann das mindestens eine digitale Lichtmodulationselement an jede Stelle der Vorrichtung eingebracht sein, an der es durch den mindestens einen aufgeweiteten, kollimierten Strahl ausreichend beleuchtet wird. In einer besonderen Ausgestaltung kann das mindestens eine digitale Lichtmodulationselement vorzugsweise in eine Ebene eines Zwischenbildes eingebracht sein. In dieser Ausgestaltung kann das mindestens eine digitale Lichtmodulationselement als Intensitätsmodulator, zwischen gekreuzten Polarisatoren, wirken.

Die hierin vorgeschlagene kompakte und robust aufgebaute Vorrichtung ermöglicht somit auf einfache Weise eine Erzeugung von isoluminanten und monochromatischen oder polychromatischen Interferenzmustern, insbesondere in Form von Interferenzstreifen in einer oder in zwei Dimensionen. Aufgrund des Einsatzes des mindestens einen digitalen Lichtmodulationselements wird hierbei insbesondere eine variable Darstellung von verschiedenen Interferenzmustern ermöglicht. Bei erfindungsgemäßer Verwendung mindestens einer Superkontinuum-Laserquelle und mindestens einem abstimmbaren Wellenlängenfilter lässt sich damit eine Wellenfront in spektral verschiedene Bereiche auf der jeweiligen Pupillenebene auflösen. Damit lässt sich in vorteilhafter Weise der Stimulus in der Eingangspupille und nicht im Zwischenbild kontrollieren. Hierdurch kann eine Überlappung in zwei konjugierten Pupillen ermöglicht werden. Für weitere Einzelheiten wird auf die Ausführungsbeispiele verwiesen.

In einem weiteren Aspekt betrifft die vorliegende Erfindung eine Vorrichtung zur Bestimmung einer neuronalen Übertragungsfunktion einer Sehbahn eines Nutzers. Die Vorrichtung zur Bestimmung einer neuronalen Übertragungsfunktion der Sehbahn des Nutzers umfasst hierbei mindestens eine oben oder unten näher beschriebene Vorrichtung zur Erzeugung von mindestens zwei Punktlichtquellen derselben Wellenlänge auf einer Pupillenebene mindestens eines Auges eines Nutzers sowie eine Auswerteeinheit, die zur Bestimmung einer neuronalen Übertragungsfunktion der Sehbahn des Nutzers aus den Interferenzmustern, die auf der Retina des mindestens einen Auges des Nutzers abgebildet werden, eingerichtet ist. Auf diese Weise kann die neuronale Übertragungsfunktion für eine beliebige Wellenlänge, für das gesamte sichtbare Spektrum oder einen Teil davon bestimmt werden.

Hierbei kann die Auswerteeinheit dazu eingerichtet sein, Informationen aus mindestens einer objektiven Reaktion des Nutzers auf die auf der Retina des mindestens einen Auges des Nutzers abgebildeten Interferenzmuster zu erzeugen, wobei die Reaktion bewusst oder bevorzugt unbewusst sein kann, wobei die neuronale Übertragungsfunktion der Sehbahn des Nutzers aus den derart erzeugten Informationen bestimmt werden kann. Alternativ oder zusätzlich kann die Vorrichtung ferner eine Eingabeeinheit umfassen, die dazu eingerichtet sein kann, eine psychophysische Reaktion des Nutzers auf die auf der Retina des mindestens einen Auges des Nutzers abgebildeten Interferenzmuster zu erfassen und an die Auswerteeinheit weiterzuleiten. Hierzu können psychophysische Algorithmen eingesetzt werden, die auf gesetzmäßigen Wechselbeziehungen zwischen einem subjektiven mentalen Erleben des Nutzers und mindestens einem quantitativ messbaren, objektiven physikalischen Stimulus in Form der auf der jeweiligen Retina des mindestens einen Auges des Nutzers abgebildeten Interferenzmusters als Auslöser für das Erleben des Nutzers basieren. Die Eingabeeinheit kann hierbei eine beliebige Einrichtung umfassen, mittels welcher ein subjektives Feedback durch den Nutzer, z.B. in Form einer manuellen, akustischen oder taktilen Eingabe, erhalten werden kann. Bei der Eingabeeinheit kann es sich um eine Tastatur, insbesondere um eine Tastatur, die über Tasten verfügt, die der Nutzer bedienen, bevorzugt drücken, kann, handeln. Vorzugsweise kann es sich, alternativ oder zusätzlich, um eine auf einem berührungsempfindlichen Bildschirm (Touchscreen) eines mobilen Kommunikationsgeräts dargestellte virtuelle Tastatur handeln, die der Nutzer ebenfalls bedienen, bevorzugt drücken, kann. Durch ein Bedienen der Eingabeeinheit kann somit der Nutzer mittels der Eingabeeinheit ein Messsignal erzeugen, das an die Auswerteeinheit weitergeleitet werden kann.

Unter einem mobilen Kommunikationsgerät ist bevorzugt eine Vorrichtung zu verstehen, welche zumindest einen programmierbaren Prozessor sowie wenigstens eine Kamera und wenigstens einen Beschleunigungssensor umfasst, und welche bevorzugt dazu ausgelegt ist, getragen zu werden, d.h. hinsichtlich Dimensionen und Gewicht derart ausgestaltet ist, dass sie von einer Person mitführbar ist. In dem wenigstens einen mobilen Kommunikationsgerät können weitere Komponenten vorhanden sein, wie zum Beispiel wenigstens ein Bildschirm, wenigstens eine Lichtquelle für beispielsweise sichtbares Licht aus einem Wellenlängenbereich von 380 nm bis 780 nm und/oder infrarotes Licht aus einem Wellenlängenbereich von 780 nm bis 1 mm und/oder wenigstens einen Lichtempfänger mit einer Sensibilität für beispielsweise sichtbares Licht aus einem Wellenlängenbereich von 380 nm bis 780 nm und/oder infrarotes Licht aus einem Wellenlängenbereich von >780 nm bis 1 mm. Typische Beispiele für derartige mobile Kommunikationsgeräte sind Smartphones oder Tablet-PCs, die wenigstens einen Bildschirm, beispielsweise wenigstens einen berührungsempfindlichen Bildschirm (Touchscreen), wenigstens eine Kamera, wenigstens einen Beschleunigungssensor, wenigstens eine Lichtquelle, wenigstens einen Lichtempfänger und weitere Komponenten, wie drahtlosen Schnittstellen für Mobilfunk oder WLAN (Wireless LAN) aufweisen können.

In einem weiteren Aspekt betrifft die vorliegende Erfindung ein Verfahren zur Erzeugung von mindestens zwei Punktlichtquellen derselben Wellenlänge auf einer Pupillenebene mindestens eines Auges eines Nutzers. Das Verfahren umfasst die folgenden Schritte, vorzugsweise in der angegebenen Reihenfolge, wobei eine ganz oder teilweise zeitgleiche Ausführung der Verfahrensschritte möglich ist. Weiterhin können einzelne, mehrere oder alle Schritte des Verfahrens wiederholt, insbesondere mehr als einmal, ausgeführt werden. Das Verfahren kann, zusätzlich zu den genannten Verfahrensschritten auch weitere Verfahrensschritte umfassen.

Das Verfahren zur Erzeugung von mindestens zwei Punktlichtquellen derselben Wellenlänge auf einer Pupillenebene mindestens eines Auges eines Nutzers, umfassend die Schritte:
a) Erzeugen mindestens eines Lichtstrahls mittels mindestens einer kohärenten Lichtquelle;
b) Aufteilen des mindestens einen Lichtstrahls jeweils in zwei Paare von Teillichtstrahlen, Überlagern der jeweiligen Teillichtstrahlen in jedem Paar und Einstellen von Kontrast und räumlicher Phase in einem aus der Überlagerung der beiden Teillichtstrahlen für jedes Paar gebildeten Interferenzmuster; und
c) Führen jedes Paares der überlagerten Teillichtstrahlen derart, dass mindestens zwei Punktlichtquellen derselben Wellenlänge auf einer bzw. der jeweiligen Pupillenebene mindestens eines Auges eines Nutzers erzeugt werden,
wobei zur Durchführung von Schritt b) mindestens ein digitales Lichtmodulationselement verwendet wird, wobei das digitale Lichtmodulationselement eine optische Vorrichtung ist, die eine Vielzahl an einzeln ansteuerbaren optischen Elementen aufweist, die zur Modulation des eintreffenden Lichtstrahls eingerichtet sind, und wobei der mindestens eine Lichtstrahl durch die kohärente Lichtquelle mittels mindestens einer polychromatischen Lichtquelle mit einer Superkontinuum-Laserquelle und mindestens einem abstimmbaren Wellenlängenfilter erzeugt wird.

Gemäß Verfahrensschritt a) erfolgt ein Erzeugen mindestens eines Lichtstrahls mittels mindestens einer kohärenten Lichtquelle.

Gemäß Verfahrensschritt b) erfolgt ein Aufteilen des mindestens einen Lichtstrahls jeweils in zwei Paare von Teillichtstrahlen, ein Überlagern der jeweiligen Teillichtstrahlen in jedem Paar und ein Einstellen von Kontrast und räumlicher Phase in einem aus der Überlagerung der beiden Teillichtstrahlen für jedes Paar gebildeten Interferenzmuster, wofür erfindungsgemäß mindestens ein digitales Lichtmodulationselement verwendet wird.

Gemäß Verfahrensschritt c) erfolgt schließlich ein Führen jedes Paares der überlagerten Teillichtstrahlen derart, dass die mindestens zwei gewünschten Punktlichtquellen derselben Wellenlänge auf der jeweiligen Pupillenebene des mindestens einen Auges des Nutzers erzeugt werden.

In einer besonders bevorzugten Ausgestaltung erfolgt das Führen jedes Paares der überlagerten Teillichtstrahlen gemäß Schritt c) derart, dass die beiden Interferenzmuster, insbesondere die jeweiligen Interferenzstreifen in einer oder in zwei Dimensionen, jeweils auf der jeweiligen Retina des mindestens einen Auges des Nutzers abgebildet werden.

In einem weiteren Aspekt betrifft die vorliegende Erfindung ein Verfahren zur Bestimmung einer neuronalen Übertragungsfunktion einer Sehbahn eines Nutzers. Das Verfahren zur Bestimmung einer neuronalen Übertragungsfunktion der Sehbahn des Nutzers umfasst die Schritte a) bis c) gemäß dem Verfahren zur Erzeugung von mindestens zwei Punktlichtquellen derselben Wellenlänge auf einer bzw. der jeweiligen Pupillenebene mindestens eines Auges eines Nutzers sowie zusätzlich einen Schritt d):
d) Bestimmen einer neuronalen Übertragungsfunktion der Sehbahn des Nutzers aus den Interferenzmustern, die auf der jeweiligen Retina des mindestens einen Auges des Nutzers abgebildet werden.

In einer besonders bevorzugten Ausgestaltung kann hierbei Schritt d) ein Erfassen einer psychophysischen Reaktion des Nutzers auf die auf der jeweiligen Retina des mindestens einen Auges des Nutzers abgebildeten Interferenzmuster umfassen. Insbesondere um eine aussagekräftige psychometrische Funktion zu bestimmen, kann es besonders vorteilhaft sein, zumindest den Schritt d) mehrfach durchzuführen, selbst dann, wenn die Schritte a) bis c) nur einfach ausgeführt werden.

Für weitere Einzelheiten in Bezug auf die vorliegenden Verfahren wird auf die übrige Beschreibung der Vorrichtung verwiesen.

In einem weiteren Aspekt betrifft die vorliegende Erfindung jeweils
- ein Computerprogramm zur Erzeugung von mindestens zwei Punktlichtquellen derselben Wellenlänge auf einer bzw. der jeweiligen Pupillenebene mindestens eines Auges eines Nutzers, und
- ein Computerprogramm zur Bestimmung einer neuronalen Übertragungsfunktion einer Sehbahn eines Nutzers,
wobei das jeweilige Computerprogramm dazu eingerichtet ist, die zugehörigen Schritte a) bis c) oder a) bis d) des betreffenden Verfahrens durchzuführen.

Für weitere Einzelheiten in Bezug auf die vorliegenden Computerprogramme wird auf die übrige Beschreibung der Verfahren und der Vorrichtungen verwiesen.

Die erfindungsgemäße Vorrichtung und die vorliegenden Verfahren weisen gegenüber herkömmlichen Vorrichtungen und Verfahren zahlreiche Vorteile auf. Die Vorrichtungen sind kompakt und robust aufgebaut und ermöglichen so eine einfache Bedienung, um in einem kommerziellen Produkt beispielsweise in einer klinischen Umgebung eingesetzt werden zu können. Diese Vorteile basieren insbesondere auf der Verwendung mindestens eines digitalen Lichtmodulationselements, vorzugsweise mindestens eines räumlichen Lichtmodulators oder mindestens einer digitalen Mikrospiegel-Einheit, und vermeiden daher eine komplizierte, aufwändige und störanfällige Kombination, umfassend einen rotierenden Würfel oder ein rotierendes Prisma. Auf diese Weise kann die gewünschte Bestimmung der neuronalen Übertragungsfunktion der Sehbahn des Nutzers, vorzugsweise über das gesamte optische Spektrum erfolgen.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:
- Figur 1: eine schematische Darstellung eines bevorzugten Ausführungsbeispiels einer Vorrichtung zur Bestimmung einer neuronalen Übertragungsfunktion einer Sehbahn eines Nutzers;
- Figur 2: eine schematische Darstellung einer Einstellung eines Kontrasts in Interferenzmustern mittels eines digitalen Lichtmodulationselements; und
- Figur 3: eine schematische Darstellung der Einstellung des digitalen Lichtmodulationselements zur Erzeugung eines polychromatischen Interferenzmusters;
- Figur 4: eine schematische Darstellung von besonders bevorzugten Interferenzmustern; und
- Figur 5: ein Ablaufdiagramm eines bevorzugten Ausführungsbeispiels eines Verfahrens zur Bestimmung der neuronalen Übertragungsfunktion einer Sehbahn des Nutzers.

### Ausführungsbeispiele

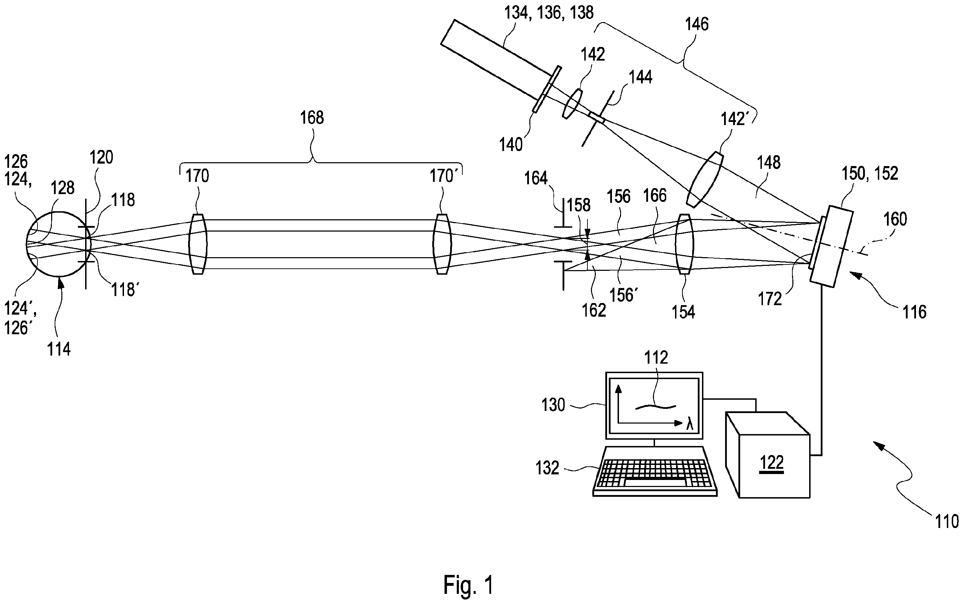

Figur 1 zeigt eine schematische Darstellung eines bevorzugten Ausführungsbeispiels einer Vorrichtung 110 zur Bestimmung einer neuronalen Übertragungsfunktion 112 einer Sehbahn eines Nutzers. Die Sehbahn bezeichnet hierbei eine Kette von Organen des Nutzers, die dazu eingerichtet ist, dem Nutzer das Sehen zu ermöglichen, wobei die Kette der Organe mindestens ein Auge 114, Retina, Sehnerv sowie alle Zentren im Gehirn, die zum Sehen beitragen, umfasst. Die Vorrichtung 110 zur Bestimmung der neuronalen Übertragungsfunktion 112 der Sehbahn des Nutzers umfasst eine Vorrichtung 116 zur Erzeugung von zwei Punktlichtquellen 118, 118' derselben Wellenlänge auf einer Pupillenebene 120 des Auges 114 des Nutzers sowie eine Auswerteeinheit 122, die zur Bestimmung der neuronalen Übertragungsfunktion 112 der Sehbahn des Nutzers aus Interferenzmustern 124, 124' eingerichtet ist, wobei die Interferenzmuster 124, 124' insbesondere in Form von Schachbrettmustern 126, 126' vorliegen, die auf der Retina 128, d.h. auf der lichtempfindlichen Schicht, des Auges 114 des Nutzers abgebildet werden. Hierbei kann die Auswerteeinheit 122 dazu eingerichtet sein, Informationen aus mindestens einer objektiven, bewussten oder bevorzugt unbewussten Reaktion des Nutzers auf die Interferenzmuster 124, 124' zu erzeugen, wobei die neuronale Übertragungsfunktion 112 der Sehbahn des Nutzers aus Informationen bestimmt werden kann. Alternativ oder zusätzlich kann die Vorrichtung 110 ferner eine Eingabeeinheit (nicht dargestellt) umfassen, die dazu eingerichtet sein kann, eine psychophysische Reaktion des Nutzers auf die Interferenzmuster 124, 124' zu erfassen und an die Auswerteeinheit 122 weiterzuleiten. Die Eingabeeinheit kann hierbei ausgewählt sein aus mindestens einer Tastatur, einer auf einem berührungsempfindlichen Bildschirm (Touchscreen) eines mobilen Kommunikationsgeräts dargestellten virtuellen Tastatur, einem Mikrofon oder einem taktilen Sensor. Andere Arten von Eingabeeinheiten sind jedoch möglich.

Wie Figur 1 zeigt, können zur Darstellung der neuronalen Übertragungsfunktion 112 der Sehbahn des Nutzers ein Monitor 130 und zur Ansteuerung sowohl des Monitors 130 als auch der Auswerteeinheit 122 eine Tastatur 132 verwendet werden. Alternativ oder zusätzlich kann die Darstellung der neuronalen Übertragungsfunktion 112 der Sehbahn des Nutzers und/oder die Ansteuerung der Auswerteeinheit 122 mittels eines mobilen Kommunikationsgeräts (nicht dargestellt), insbesondere mittels eines Smartphones oder eines Tablets, erfolgen. Andere Arten von mobilen Kommunikationsgeräten sind jedoch möglich. Als weitere Alternative kann das mobile Kommunikationsgerät selbst als die Auswerteeinheit 122 ausgestaltet sein.

Die Vorrichtung 116 zur Erzeugung der beiden Punktlichtquellen 118, 118' derselben Wellenlänge auf der Pupillenebene 120 des Auges 114 des Nutzers umfasst eine kohärente Lichtquelle 134, die als abstimmbare polychromatische Lichtquelle 136 vorliegt. Die polychromatische Lichtquelle umfasst eine Superkontinuum-Laserquelle 138, die kohärentes Laserlicht mehrerer Wellenlängen bereitstellt, wobei ein abstimmbares Wellenlängenfilter 140 zur Auswahl eines schmalbandigen Bereichs aus dem breitbandigen Bereich eingesetzt wird. Die Kombination aus der Superkontinuum-Laserquelle 138 und dem abstimmbaren Wellenlängenfilter 140 ermöglicht eine schnelle Bestimmung der neuronalen Übertragungsfunktion 112 über das gesamte sichtbare Spektrum oder einen Teil davon.

Das in Figur 1 schematisch dargestellte Ausführungsbeispiel der Vorrichtung 116 zur Erzeugung der beiden Punktlichtquellen 118, 118' derselben Wellenlänge auf der Pupillenebene 120 des Auges 114 des Nutzers umfasst weiterhin einen zwischen zwei optischen Linsen 142, 142' angeordneten Diffusor 144, die gemeinsam als strahlaufweitendes, kollimierendes optisches Element 146 dafür sorgen, dass der von der kohärenten Lichtquelle 134 bereitgestellte Lichtstrahl 148 aufgeweitet und kollimiert wird, bevor ein digitales Lichtmodulationselement 150 beaufschlagt. Der Diffusor 144 ermöglicht es insbesondere, eine Sichtbarkeit von Specklemustern, die durch das kohärente Licht erzeugt werden, zu verringern, bevorzugt um eine Messgenauigkeit bei geringen Kontrasten zu erhöhen.

Der aufgeweitete und kollimierte Lichtstrahl 148 beaufschlagt somit das von der Vorrichtung 116 zur Erzeugung der beiden Punktlichtquellen 118, 118' derselben Wellenlänge auf der Pupillenebene 120 des Auges 114 des Nutzers weiterhin umfasste digitale Lichtmodulationselement 150. Das in dem Ausführungsbeispiel gemäß Figur 1 verwendete digitale Lichtmodulationselement 148 ist als räumlicher Lichtmodulator 152 (engl. "Spatial Light Modulator" oder kurz "SLM") ausgeführt, der zur elektronischen und/oder optischen Aufprägung eines Intensitätsmusters, insbesondere in Form einer räumlichen Modulation und/oder einer Phase, auf den eintreffenden Lichtstrahl eingerichtet ist. Alternativ kann das digitale Lichtmodulationselement 150 auch als digitale Mikrospiegel-Einheit (engl. "Digital Micromirror Device", kurz "DMD"; nicht dargestellt) ausgeführt sein.

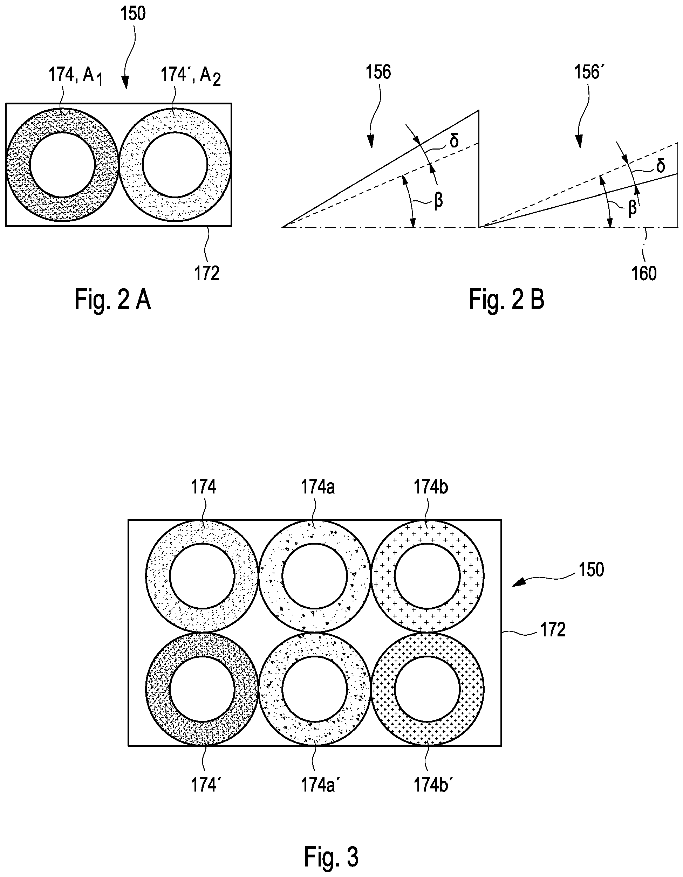

Das digitale Lichtmodulationselement 150 ist, im vorliegenden Ausführungsbeispiel durch eine optische Linse 154 unterstützt, erfindungsgemäß dazu eingerichtet, den aufgeweiteten und kollimierten Lichtstrahl 148 in zwei Paare von Teillichtstrahlen 156, 156' derart aufzuteilen, dass sich die jeweiligen Teillichtstrahlen 156, 156' in jedem Paar derart überlagern, dass sich die zur Abbildung auf der Retina 128 vorgesehenen Interferenzmuster 124, 124' ausbilden. Hierbei kann das digitale Lichtmodulationselement 150 in vorteilhafter Weise gleichzeitig dafür eingerichtet sein, Kontrast und räumliche Phase in den aus der Überlagerung der beiden Teillichtstrahlen 156, 156' für jedes Paar gebildeten Interferenzmustern 124, 124' einzustellen. Wie Figur 1 weiterhin zeigt, kann das digitale Lichtmodulationselement 150 dazu eingerichtet sein, eine laterale Verschiebung 158 der Ausbreitungsrichtung eines ersten Paars der Teillichtstrahlen 156 in Bezug auf die Ausbreitungsrichtung des anderen Paars der Teillichtstrahlen 156' vorzunehmen. Dadurch kann das digitale Lichtmodulationselement 150 den eintreffenden Lichtstrahl 148 in zwei symmetrisch zueinander geneigte Paare von Teillichtstrahlen 156, 156' aufteilen. Für weitere Einzelheiten hierzu wird auf die Beschreibung zu den Figuren 2A und 3 verwiesen. Zur Ansteuerung des digitalen Lichtmodulationselements 150, insbesondere zur Einstellung des Kontrastes und der räumlichen Phase in den Interferenzmustern 124, 124', kann eine eigene Ansteuereinheit oder, wie in Figur 1 schematisch dargestellt, die oben erwähnte Auswerteeinheit 122 verwendet werden.

Wie weiterhin aus Figur 1 hervorgeht, kann das digitale Lichtmodulationselement 150 weiterhin dazu eingerichtet sein, den eintreffenden Lichtstrahl 148 derart aufzuteilen, dass die beiden Paare der Teillichtstrahlen 156, 156' eine gemeinsame Neigung β aufweisen. In dieser Ausführung kann eine optische Achse 160 des digitalen Lichtmodulationselements 150 derart ausgerichtet sein, dass auf das digitale Lichtmodulationselement 150 auftreffendes Licht 162, das jedoch nicht durch das digitale Lichtmodulationselement 150 moduliert wurde, mittels eines optischen Filters, vorzugsweise mittels einer Apertur-Blende 164, aus dem Lichtstrahl 166, der sich nach dem Durchqueren des digitalen Lichtmodulationselements 150 ausbildet, entfernt werden kann.

In einer bevorzugten Ausführung kann eine Computereinheit (nicht dargestellt) verwendet werden, welche die Auswerteeinheit 122, den Monitor 130 und die Tastatur 132 umfasst und die zur Ansteuerung des digitalen Lichtmodulationselements 150 und zur Erzeugung der Interferenzmuster 124, 124' auf der Retina 128 des Auges 114 des Nutzers eingerichtet sein kann. Dieselbe Computereinheit kann hierbei zusätzlich zur Aufnahme von optischen Messsignalen und/oder zur Aufnahme von subjektiven psychophysischen Signalen, die aus einer Reaktion des Nutzers erzeugt werden können, und/oder zur Bestimmung der neuronalen Übertragungsfunktion 112 eingerichtet sein. Alternativ oder zusätzlich können hierfür eine oder mehrere individuelle Computereinheiten eingesetzt werden.

Wie Figur 1 weiterhin zeigt, wird jedes Paar der überlagerten Teillichtstrahlen 156, 156' mittels eines dafür eingerichteten Strahlengangs 168, der zwei weitere optische Linsen 170, 170' umfasst, derart in Richtung des Auges 114 des Nutzers geführt, dass schließlich die beiden gewünschten Punktlichtquellen 118, 118' derselben Wellenlänge auf der Pupillenebene 120 des Auges 114 des Nutzers erzeugt werden. In der Ausführung gemäß Figur 1 ist der Strahlengang 168 derart eingerichtet, dass die beiden Interferenzmuster 124, 124' jeweils auf der Retina 128 des Auges 114 des Nutzers abgebildet werden.

Figur 2 zeigt eine schematische Darstellung der Einstellung des Kontrasts in den Interferenzmustern 124, 124' mittels des digitalen Lichtmodulationselements 150.

Hierzu sind in Figur 2A auf einer Ebene 172 des digitalen Lichtmodulationselements 150 dem jeweiligen Paar der Teillichtstrahlen 156, 156' zugeordnete Ringe 174, 174' schematisch dargestellt. Ein Verhältnis der Flächen A₁:A₂ der Ringe 174, 174' kann hierbei eine Einstellung des Kontrasts des zugehörigen Interferenzmuster 124, 124', insbesondere der zugehörigen Schachbrettmuster 126, 126', auf der Retina 128 des Auges 114 des Nutzers ermöglichen. Beispielsweise kann durch Einstellung des Verhältnisses der Flächen A₁:A₂ = 1:1 eine isoluminante Einstellung des Kontrasts von 100 % in den Interferenzmustern 124, 124' erfolgen. Dagegen kann durch Einstellung des Verhältnisses der Flächen A₁:A₂ = 4:1 oder A₁:A₂ = 1:4 ein Kontrast von 25 % erzeugt werden, während durch Einstellung des Verhältnisses der Flächen A₁:A₂ = 1:0 oder A₁:A₂ = 0:1 ein Kontrast von 0 % erzeugt werden kann. Weiter Beispiele sind möglich. Ein derartiges Vorgehen kann insbesondere eine Vornahme von Kontraständerungen in den Interferenzmustern 124, 124' mit einer hohen Wiederholrate, die lediglich durch die Wiederholrate des digitalen Lichtmodulationselements 150 begrenzt ist, ermöglichen, während zugleich eine Leuchtdichte der beiden Teillichtstahlen 156, 156' konstant gehalten werden kann.

Figur 2B zeigt eine schematische Darstellung von Neigungen des Lichtstrahls 166, der sich nach dem Durchqueren des digitalen Lichtmodulationselements 150 ausbildet, gegenüber der optischen Achse 160 des digitalen Lichtmodulationselements 150. Zusätzlich zu einer gemeinsamen Neigung β, über welche die beiden Paare der Teillichtstrahlen 156, 156' verfügen, kann jeder der Teillichtstrahlen 156, 156' in Folge der Flächen A1, A2 der Ringe 174, 174' auf der Ebene 172 des digitalen Lichtmodulationselements 150 eine individuelle Neigung δ, δ` aufweisen.

Die Vorrichtung 116 zur Erzeugung der beiden Punktlichtquellen 118, 118' derselben Wellenlänge auf der Pupillenebene 120 des Auges 114 des Nutzers ermöglicht somit die Erzeugung von isoluminanten monochromatischen Interferenzmustern 124, 124', die insbesondere als Schachbrettmuster 126, 126' vorliegen können. Zur Erzeugung von polychromatischen Interferenzmustern (nicht dargestellt), insbesondere von polychromatischen Schachbrettmustern, wird die oben erwähnte abstimmbare polychromatische Lichtquelle 136 mit der Superkontinuum-Laserquelle 138 in Kombination mit dem abstimmbaren Wellenlängenfilter 140 eingesetzt, um aus bereitgestelltem kohärentem Laserlicht mehrerer Wellenlängen jeweils einen einer bestimmten Farbe zugeordneten schmalbandigen Bereich auszuwählen, um damit eine Wellenfront in spektral verschiedene Bereiche auf der Pupillenebene 120 auflösen.

Hierbei können zwei individuelle schmalbandige Bereiche ausgewählt werden, z.B. aus dem grünen und dem roten Spektralbereich. Auf diese Weise können vier Ringe (nicht dargestellt) auf der Ebene 172 des digitalen Lichtmodulationselements 150 erzeugt werden, jeweils ein Paar für jede Farbe. Eine Einstellung der relativen Positionen der grünen und der roten Interferenzmuster 124, 124' kann durch Einführung einer konstanten Phasenverschiebung in demjenigen Teil des digitalen Lichtmodulationselements 150 ermöglicht werden, welcher einer der Farben auf der die zugehörige Neigung δ, δ` bestimmenden Ortsfrequenz entspricht.

Figur 3 zeigt eine schematische Darstellung eine schematische Darstellung der Einstellung des digitalen Lichtmodulationselements zur Erzeugung eines polychromatischen Interferenzmusters für drei individuelle schmalbandige Bereiche, z.B. aus dem blauen, dem grünen und dem roten Spektralbereich. Auf diese Weise können sechs Ringe 174, 174', 174a, 174a', 174b, 174b' auf der Ebene 172 des digitalen Lichtmodulationselements 150 erzeugt werden, jeweils ein Paar für jede Farbe, wobei jedes Paar seiner Wellenlänge entspricht. Hierbei kann vorzugsweise für jedes Paar die zugehörige Neigung δ, δ`, δ" (nicht dargestellt) einzeln ermittelt werden, da die jeweilige Phasenverschiebung von der betreffenden Wellenlänge abhängt, um jeweilige Interferenzstreifen in den Schachbrettmustern 126, 126' mit derselben Periode zu erzeugen. Auf diese Weise kann ein stabiles polychromatisches Interferenzmuster erzeugt werden, das auf der Retina abgebildet wird.

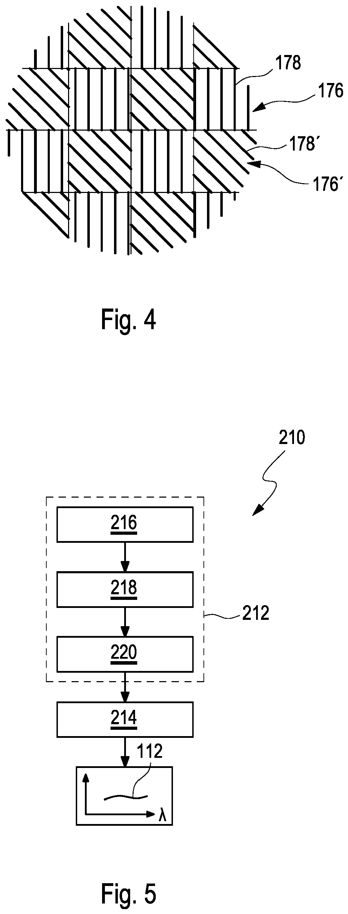

Figur 4 zeigt eine schematische Darstellung einer besonders bevorzugten Ausführung der zur Abbildung auf der Retina 128 vorgesehenen Interferenzmuster 124, 124', die, wie hier dargestellt, in Form des Schachbrettmusters 126 vorliegen, das erste Bereiche 176 und zweite Bereiche 176' aufweist. Hierbei verfügt jeder der beiden Bereiche 176, 176' jeweils über Interferenzstreifen 178, 178', wobei sich die beide Bereiche 176, 176' durch die jeweilige Neigung δ, δ` der Interferenzstreifen 178, 178' voneinander unterscheiden. Eine Erzeugung dieser Art von Interferenzmustern 124, 124' kann vorzugsweise mittels einer Phasenmaske auf dem digitalen Lichtmodulationselement 150, insbesondere auf dem räumlichen Lichtmodulator 152, erfolgen, insbesondere durch Kombination zweier maskierter Pupillen in Form des Schachbrettmusters 126 mit zwei simultan applizierten Neigungen δ, δ` der Interferenzstreifen 178, 178'. Weitere Ausführungen der zur Abbildung auf der Retina 128 vorgesehenen Interferenzmuster 124, 124' sind jedoch denkbar.

Figur 5 zeigt ein Ablaufdiagramm eines bevorzugten Ausführungsbeispiels eines Verfahrens 210 zur Bestimmung der neuronalen Übertragungsfunktion 112 der Sehbahn des Nutzers.

Das Verfahren 210 zur Bestimmung der neuronalen Übertragungsfunktion 112 der Sehbahn des Nutzers umfasst hierbei ein Verfahren 212 zur Erzeugung der beiden Punktlichtquellen 118, 118' derselben Wellenlänge auf der Pupillenebene 120 des Auges 114 des Nutzers sowie einen Bestimmungsschritt 214 gemäß Schritt d) zum Bestimmen der neuronalen Übertragungsfunktion 112 der Sehbahn des Nutzers aus den Interferenzmustern 124, 124', insbesondere den in Figur 4 schematisch dargestellten Schachbrettmustern 126, 126',die erste Bereiche 176 und zweite Bereiche 176' aufweisen können, die jeweils die Interferenzstreifen 178, 178' mit unterschiedlicher Neigung δ, δ` umfassen, die auf der Retina 128 des Auges 114 des Nutzers abgebildet werden.

Um die Interferenzmuster 124, 124', insbesondere die Schachbrettmuster 126, 126', die auf der Retina 128 des Auges 114 des Nutzers abgebildet werden, zu erzeugen, erfolgt
- in einem Beleuchtungsschritt 216 gemäß Schritt a) ein Erzeugen des Lichtstrahls 148 mittels der kohärenten Lichtquelle 134, d.h. mittels der abstimmbaren polychromatische Lichtquelle 136 mit der Superkontinuum-Laserquelle 138;
- in einem Modulationsschritt 218 unter Verwendung des digitalen Lichtmodulationselements 150, insbesondere des räumlichen Lichtmodulators 152, gemäß Schritt b) ein Aufteilen des Lichtstrahls 148 in zwei Paare von Teillichtstrahlen 156, 156', ein Überlagern der jeweiligen Teillichtstrahlen 156, 156' in jedem Paar und ein Einstellen von Kontrast und räumlicher Phase in einem aus der Überlagerung der beiden Teillichtstrahlen 156, 156' für jedes Paar gebildeten Interferenzmuster 124, 124', und
- in einem Beaufschlagungsschritt 220 gemäß Schritt c) ein Führen jedes Paares der überlagerten Teillichtstrahlen 156, 156' derart, dass die beiden Punktlichtquellen 118, 118' derselben Wellenlänge derart auf der Pupillenebene 120 des Auges 114 eines Nutzers erzeugt werden, dass die beiden Interferenzmuster 124, 124' jeweils auf der Retina 128 des Auges 114 des Nutzers abgebildet werden.

### Bezugszeichenliste

- 110: Vorrichtung zur Bestimmung der neuronalen Übertragungsfunktion einer Sehbahn eines Nutzers
- 112: neuronale Übertragungsfunktion
- 114: Auge
- 116: Vorrichtung zur Erzeugung der beiden Punktlichtquellen derselben Wellenlänge auf der Pupillenebene mindestens eines Auges eines Nutzers
- 118, 118': Punktlichtquelle
- 120: Pupillenebene
- 122: Auswerteeinheit
- 124, 124': Interferenzmuster
- 126, 126': Schachbrettmuster
- 128: Retina (Netzhaut)
- 130: Monitor
- 132: Tastatur
- 134: kohärente Lichtquelle
- 136: polychromatische Lichtquelle
- 138: Superkontinuum-Laserquelle
- 140: abstimmbares Wellenlängenfilter
- 142, 142': optischen Linse
- 144: Diffusor
- 146: strahlaufweitendes, kollimierendes optisches Element
- 148: Lichtstrahl
- 150: digitales Lichtmodulationselement
- 152: räumlicher Lichtmodulator
- 154: optische Linse
- 156, 156': Paar von Teillichtstrahlen
- 158: laterale Verschiebung
- 160: optische Achse
- 162: nicht-moduliertes Licht
- 164: Apertur-Blende
- 166: Lichtstrahl
- 168: Strahlengang
- 170, 170': weitere optische Linsen
- 172: Ebene (des digitalen Lichtmodulationselements)
- 174, 174' ...: Ring
- 176, 176': Bereich
- 178, 178': Interferenzstreifen
- 210: Verfahren zur Bestimmung einer neuronalen Übertragungsfunktion einer Sehbahn eines Nutzers
- 212: Verfahren zur Erzeugung von zwei Punktlichtquellen derselben Wellenlänge auf einer Pupillenebene mindestens einen Auges eines Nutzers
- 214: Bestimmungsschritt
- 216: Beleuchtungsschritt
- 218: Modulationsschritt
- 220: Beaufschlagungsschritt

## Patentansprüche

1. Vorrichtung (116) zur Erzeugung von mindestens zwei Punktlichtquellen (118, 118') derselben Wellenlänge auf einer Pupillenebene (120) mindestens eines Auges (114) eines Nutzers, umfassend
- mindestens eine kohärente Lichtquelle (134) zur Erzeugung mindestens eines Lichtstrahls (148);
- mindestens eine optische Einrichtung zur Aufteilung des mindestens einen Lichtstrahls (148) jeweils in zwei Paare von Teillichtstrahlen (156, 156'), zur Überlagerung der jeweiligen Teillichtstrahlen (156, 156') in jedem Paar und zur Einstellung von Kontrast und räumlicher Phase in einem aus der Überlagerung der beiden Teillichtstrahlen (156, 156') für jedes Paar gebildeten Interferenzmusters (124, 124'), wobei die mindestens eine optische Einrichtung mindestens ein digitales Lichtmodulationselement (150) aufweist, wobei das digitale Lichtmodulationselement (150) eine optische Vorrichtung ist, die eine Vielzahl an einzeln ansteuerbaren optischen Elementen aufweist, die zur Modulation des eintreffenden Lichtstrahls (148) eingerichtet sind; und
- mindestens einen Strahlengang (168) zur Führung jedes Paares der überlagerten Teillichtstrahlen (156, 156') derart, dass mindestens zwei Punktlichtquellen (118, 118') derselben Wellenlänge auf der jeweiligen Pupillenebene (120) mindestens eines Auges (114) eines Nutzers erzeugt werden;
**dadurch gekennzeichnet,**
**dass** die mindestens eine kohärente Lichtquelle (134) mindestens eine polychromatische Lichtquelle (136) mit einer Superkontinuum-Laserquelle (138) und mindestens ein abstimmbares Wellenlängenfilter (140) umfasst.

2. Vorrichtung (116) nach dem vorangehenden Anspruch, **dadurch gekennzeichnet, dass** der mindestens eine Strahlengang (168) zur Führung jedes Paares der überlagerten Teillichtstrahlen (156, 156') ferner derart eingerichtet ist, dass mindestens die beiden Interferenzmuster (124, 124') jeweils auf der jeweiligen Retina (128) des mindestens einen Auges (114) des Nutzers abgebildet werden.

3. Vorrichtung (116) nach einem der vorangehenden Ansprüche, **dadurch gekennzeichnet, dass** das mindestens eine digitale Lichtmodulationselement (150) ausgewählt ist aus mindestens einem räumlichen Lichtmodulator (152) oder mindestens einer digitalen Mikrospiegel-Einheit.

4. Vorrichtung (116) nach einem der vorangehenden Ansprüche, **dadurch gekennzeichnet, dass** das mindestens eine digitale Lichtmodulationselement (150) dazu eingerichtet ist, mindestens eine laterale Verschiebung der Teillichtstrahlen (156, 156') mindestens eines der Paare der Teillichtstrahlen (156, 156') in Bezug auf eine Ausbreitungsrichtung der Teillichtstrahlen (156, 156') mindestens eines anderen der Paare der Teillichtstrahlen (156, 156') vorzunehmen.

5. Vorrichtung (116) nach einem der vorangehenden Ansprüche, **dadurch gekennzeichnet, dass** das mindestens eine digitale Lichtmodulationselement (150) dazu eingerichtet ist, eine beliebige Einstellung des Kontrasts in dem für jedes Paar gebildeten Interferenzmuster (124, 124') vorzunehmen.

6. Vorrichtung (116) nach einem der vorangehenden Ansprüche, weiterhin umfassend mindestens ein optisches Filter, das zur Entfernung von nicht-moduliertem Licht (162) aus dem Lichtstrahl (166) nach dem Durchqueren des mindestens einen digitalen Lichtmodulationselements (150) eingerichtet ist.

7. Vorrichtung (116) nach einem der vorangehenden Ansprüche, **dadurch gekennzeichnet, dass** mindestens ein strahlaufweitendes, kollimierendes optisches System (146) zwischen der mindestens einen kohärenten Lichtquelle (134) und dem mindestens einen digitalen Lichtmodulationselement (150) angeordnet ist.

8. Vorrichtung (116) nach einem der vorangehenden Ansprüche, **dadurch gekennzeichnet, dass** die Vorrichtung (116) zur Erzeugung von jeweils mindestens zwei Punktlichtquellen (118, 118') bei jeweils einer voneinander verschiedenen Wellenlänge auf der jeweiligen Pupillenebene (120) des mindestens einen Auges (114) des Nutzers eingerichtet ist.

9. Verfahren (212) zur Erzeugung von mindestens zwei Punktlichtquellen (118, 118') derselben Wellenlänge auf der jeweiligen Pupillenebene (120) mindestens eines Auges (114) eines Nutzers, umfassend die Schritte:
a) Erzeugen mindestens eines Lichtstrahls (148) mittels mindestens einer kohärenten Lichtquelle (134);
b) Aufteilen des mindestens einen Lichtstrahls (148) jeweils in zwei Paare von Teillichtstrahlen (156, 156'), Überlagern der jeweiligen Teillichtstrahlen (156, 156') in jedem Paar und Einstellen von Kontrast und räumlicher Phase in einem aus der Überlagerung der beiden Teillichtstrahlen (156, 156') für jedes Paar gebildeten Interferenzmuster (124, 124') mittels mindestens einem digitalen Lichtmodulationselement (150), wobei das digitale Lichtmodulationselement (150) eine optische Vorrichtung ist, die eine Vielzahl an einzeln ansteuerbaren optischen Elementen aufweist, die zur Modulation des eintreffenden Lichtstrahls (148) eingerichtet sind; und
c) Führen jedes Paares der überlagerten Teillichtstrahlen (156, 156') derart, dass mindestens zwei Punktlichtquellen (118, 118') derselben Wellenlänge auf der jeweiligen Pupillenebene (120) mindestens eines Auges (114) eines Nutzers erzeugt werden,
**dadurch gekennzeichnet,**
**dass** der mindestens eine Lichtstrahl (148) durch die kohärente Lichtquelle (134) mittels mindestens einer polychromatischen Lichtquelle (136) mit einer Superkontinuum-Laserquelle (138) und mindestens einem abstimmbaren Wellenlängenfilter (140) erzeugt wird.

10. Verfahren (212) nach dem vorangehenden Anspruch, **dadurch gekennzeichnet, dass** das Führen jedes Paares der überlagerten Teillichtstrahlen (156, 156') gemäß Schritt c) derart erfolgt, dass die beiden Interferenzmuster (124, 124') jeweils auf der jeweiligen Retina (128) des mindestens einen Auges (114) des Nutzers abgebildet werden.

11. Computerprogramm zur Erzeugung von mindestens zwei Punktlichtquellen (118, 118') derselben Wellenlänge auf der jeweiligen Pupillenebene (120) mindestens eines Auges (114) eines Nutzers, umfassend Befehle, die bei der Ausführung des Programms durch einen Computer diesen veranlassen, die folgenden Schritte mittels der Vorrichtung (116) gemäß des Anspruchs 1 durchzuführen:
a) Erzeugen mindestens eines Lichtstrahls (148) mittels der mindestens einen kohärenten Lichtquelle (134);
b) Aufteilen des mindestens einen Lichtstrahls (148) in zwei Paare von Teillichtstrahlen (156, 156') mittels der mindestens einen optischen Einrichtung, Überlagern der jeweiligen Teillichtstrahlen (156, 156') in jedem Paar und Einstellen von Kontrast und räumlicher Phase in einem aus der Überlagerung der beiden Teillichtstrahlen (156, 156') für jedes Paar gebildeten Interferenzmuster (124, 124') mittels des mindestens einen digitalen Lichtmodulationselement (150), wobei das digitale Lichtmodulationselement (150) eine optische Vorrichtung ist, die eine Vielzahl an einzeln ansteuerbaren optischen Elementen aufweist, die zur Modulation des eintreffenden Lichtstrahls (148) eingerichtet sind; und
c) Führen jedes Paares der überlagerten Teillichtstrahlen (156, 156') derart, dass mindestens zwei Punktlichtquellen (118, 118') derselben Wellenlänge auf der jeweiligen Pupillenebene (120) mindestens eines Auges (114) eines Nutzers erzeugt werden,
**dadurch gekennzeichnet,**
**dass** der mindestens eine Lichtstrahl (148) durch die kohärente Lichtquelle (134) mittels mindestens einer polychromatischen Lichtquelle (136) mit einer Superkontinuum-Laserquelle (138) und mindestens einem abstimmbaren Wellenlängenfilter (140) erzeugt wird.

## Claims

1. Apparatus (116) for producing at least two point light sources (118, 118') of the same wavelength on a pupil plane (120) of at least one eye (114) of a user, comprising
- at least one coherent light source (134) for producing at least one light beam (148);
- at least one optical device for splitting the at least one light beam (148) into two pairs of partial light beams (156, 156') in each case, for superposing the respective partial light beams (156, 156') in each pair, and for adjusting contrast and spatial phase in an interference pattern (124, 124') formed for each pair from the superposition of the two partial light beams (156, 156'), wherein the at least one optical device comprises at least one digital light modulation element (150), wherein the digital light modulation element (150) is an optical apparatus which comprises a multiplicity of individually controllable optical elements that are configured to modulate the incident light beam (148); and
- at least one beam path (168) for guiding each pair of superposed partial light beams (156, 156') such that at least two point light sources (118, 118') of the same wavelength are produced on the respective pupil plane (120) of at least one eye (114) of a user;
**characterized**
**in that** the at least one coherent light source (134) comprises at least one polychromatic light source (136) having a supercontinuum laser source (138) and comprises at least one tunable wavelength filter (140) .

2. Apparatus (116) according to the preceding claim, **characterized in that** the at least one beam path (168) for guiding each pair of superposed partial light beams (156, 156') is further configured such that at least the two interference patterns (124, 124') are each imaged on the respective retina (128) of the at least one eye (114) of the user.

3. Apparatus (116) according to either of the preceding claims, **characterized in that** the at least one digital light modulation element (150) is selected from at least one spatial light modulator (152) or at least one digital micromirror device.

4. Apparatus (116) according to any one of the preceding claims, **characterized in that** the at least one digital light modulation element (150) is configured to implement at least one lateral displacement of the partial light beams (156, 156') of at least one of the pairs of partial light beams (156, 156') in relation to a propagation direction of the partial light beams (156, 156') of at least one other pair of partial light beams (156, 156').

5. Apparatus (116) according to any one of the preceding claims, **characterized in that** the at least one digital light modulation element (150) is configured to carry out any desired adjustment of the contrast in the interference pattern (124, 124') formed for each pair.

6. Apparatus (116) according to any one of the preceding claims, furthermore comprising at least one optical filter which is configured to remove non-modulated light (162) from the light beam (166) following the passage through the at least one digital light modulation element (150).

7. Apparatus (116) according to any one of the preceding claims, **characterized in that** at least one beam-expanding, collimating optical system (146) is arranged between the at least one coherent light source (134) and the at least one digital light modulation element (150).

8. Apparatus (116) according to any one of the preceding claims, **characterized in that** the apparatus (116) is configured to produce in each case at least two point light sources (118, 118') with in each case mutually different wavelengths on the respective pupil plane (120) of the at least one eye (114) of the user.

9. Method (212) for producing at least two point light sources (118, 118') of the same wavelength on the respective pupil plane (120) of at least one eye (114) of a user, comprising the following steps:
a) producing at least one light beam (148) by means of at least one coherent light source (134);
b) splitting the at least one light beam (148) into two pairs of partial light beams (156, 156') in each case, superposing the respective partial light beams (156, 156') in each pair, and adjusting contrast and spatial phase in an interference pattern (124, 124') formed for each pair from the superposition of the two partial light beams (156, 156') by means of at least one digital light modulation element (150), wherein the digital light modulation element (150) is an optical apparatus which comprises a multiplicity of individually controllable optical elements that are configured to modulate the incident light beam (148); and
c) guiding each pair of superposed partial light beams (156, 156') such that at least two point light sources (118, 118') of the same wavelength are produced on the respective pupil plane (120) of at least one eye (114) of a user,
**characterized**
**in that** the at least one light beam (148) is produced by the coherent light source (134) by means of at least one polychromatic light source (136) having a supercontinuum laser source (138) and by means of at least one tunable wavelength filter (140).

10. Method (212) according to the preceding claim, **characterized in that** the guiding of each pair of superposed partial light beams (156, 156') in accordance with step c) is implemented in such a way that the two interference patterns (124, 124') are each imaged on the respective retina (128) of the at least one eye (114) of the user.

11. Computer program for producing at least two point light sources (118, 118') of the same wavelength on the respective pupil plane (120) of at least one eye (114) of a user, the computer program comprising commands which, when the program is executed by a computer, cause said computer to carry out the following steps by means of the apparatus (116) according to Claim 1:
a) producing at least one light beam (148) by means of the at least one coherent light source (134);
b) splitting the at least one light beam (148) into two pairs of partial light beams (156, 156') by means of the at least one optical device, superposing the respective partial light beams (156, 156') in each pair, and adjusting contrast and spatial phase in an interference pattern (124, 124') formed for each pair from the superposition of the two partial light beams (156, 156') by means of the at least one digital light modulation element (150), wherein the digital light modulation element (150) is an optical apparatus which comprises a multiplicity of individually controllable optical elements that are configured to modulate the incident light beam (148); and
c) guiding each pair of superposed partial light beams (156, 156') such that at least two point light sources (118, 118') of the same wavelength are produced on the respective pupil plane (120) of at least one eye (114) of a user,
**characterized**
**in that** the at least one light beam (148) is produced by the coherent light source (134) by means of at least one polychromatic light source (136) having a supercontinuum laser source (138) and by means of at least one tunable wavelength filter (140).

## Revendications

1. Dispositif (116) pour générer au moins deux sources lumineuses ponctuelles (118, 118') de même longueur d'onde sur un plan de pupille (120) d'au moins un œil (114) d'un utilisateur, comprenant
- au moins une source lumineuse cohérente (134) pour générer au moins un faisceau lumineux (148) ;
- au moins un dispositif optique pour séparer l'au moins un faisceau lumineux (148) respectivement en deux paires de sous-faisceaux lumineux (156, 156'), pour superposer les sous-faisceaux lumineux (156, 156') respectifs de chaque paire et pour régler le contraste et la phase spatiale dans un motif d'interférence (124, 124') formé pour chaque paire par la superposition des deux sous-faisceaux lumineux (156, 156'), dans lequel l'au moins un dispositif optique comporte au moins un élément numérique de modulation de lumière (150), dans lequel l'élément numérique de modulation de lumière (150) est un dispositif optique qui comporte une pluralité d'éléments optiques pouvant être commandés individuellement et conçus pour moduler le faisceau lumineux entrant (148) ; et
- au moins un trajet de faisceau (168) destiné à guider chaque paire des sous-faisceaux lumineux (156, 156') superposés afin de générer au moins deux sources lumineuses ponctuelles (118, 118') présentant la même longueur d'onde sur le plan de pupille (120) respectif d'au moins un œil (114) d'un utilisateur ;
**caractérisé en ce que**
l'au moins une source lumineuse cohérente (134) comprend au moins une source lumineuse polychromatique (136) dotée d'une source laser supercontinuum (138) et d'au moins un filtre de longueur d'onde accordable (140).

2. Dispositif (116) selon la revendication précédente, **caractérisé en ce que** l'au moins un trajet de faisceau (168) destiné à guider chaque paire desdits sous-faisceaux lumineux (156, 156') superposés est en outre conçu de telle sorte qu'au moins les deux motifs d'interférence (124, 124') soient respectivement soumis à une formation d'image sur la rétine (128) respective de l'au moins un œil (114) de l'utilisateur.

3. Dispositif (116) selon l'une quelconque des revendications précédentes, **caractérisé en ce que** l'au moins un élément numérique de modulation de lumière (150) est choisi parmi au moins un modulateur spatial de lumière (152) ou au moins une unité numérique à micro-miroirs.

4. Dispositif (116) selon l'une quelconque des revendications précédentes, **caractérisé en ce que** l'au moins un élément numérique de modulation de lumière (150) est conçu pour effectuer au moins un décalage latéral des sous-faisceaux lumineux (156, 156') d'au moins l'une des paires des sous-faisceaux lumineux (156, 156') par rapport à une direction de propagation desdits sous-faisceaux lumineux (156, 156') d'au moins une autre des paires des sous-faisceaux lumineux (156, 156').

5. Dispositif (116) selon l'une quelconque des revendications précédentes, **caractérisé en ce que** l'au moins un élément numérique de modulation de lumière (150) est conçu pour effectuer un réglage quelconque du contraste dans le motif d'interférence (124, 124') formé pour chaque paire.

6. Dispositif (116) selon l'une quelconque des revendications précédentes, comprenant en outre au moins un filtre optique qui est conçu pour éliminer la lumière non modulée (162) du faisceau lumineux (166) après qu'il a traversé l'au moins un élément numérique de modulation de lumière (150).

7. Dispositif (116) selon l'une quelconque des revendications précédentes, **caractérisé en ce qu'**au moins un système optique collimateur (146) d'élargissement de faisceau est disposé entre l'au moins une source lumineuse cohérente (134) et l'au moins un élément numérique de modulation de lumière (150).

8. Dispositif (116) selon l'une quelconque des revendications précédentes, **caractérisé en ce que** le dispositif (116) est conçu pour générer respectivement au moins deux sources lumineuses ponctuelles (118, 118') à des longueurs d'onde respectives différentes l'une de l'autre sur le plan de pupille (120) respectif de l'au moins un œil (114) de l'utilisateur.

9. Procédé (212) pour générer au moins deux sources lumineuses ponctuelles (118, 118') de même longueur d'onde sur le plan de pupille (120) respectif d'au moins un œil (114) d'un utilisateur, comprenant les étapes suivantes :
a) générer au moins un faisceau lumineux (148) au moyen d'au moins une source lumineuse cohérente (134) ;
b) séparer l'au moins un faisceau lumineux (148) respectivement en deux paires de sous-faisceaux lumineux (156, 156'), superposer les sous-faisceaux lumineux (156, 156') respectifs de chaque paire et régler le contraste et la phase spatiale dans un motif d'interférence (124, 124') formé pour chaque paire par la superposition des deux sous-faisceaux lumineux (156, 156') au moyen d'au moins un élément numérique de modulation de lumière (150), dans lequel l'élément numérique de modulation de lumière (150) est un dispositif optique qui comporte une pluralité d'éléments optiques pouvant être commandés individuellement et conçus pour moduler le faisceau lumineux entrant (148) ; et
c) guider chaque paire desdits sous-faisceaux lumineux (156, 156') superposés afin de générer au moins deux sources lumineuses ponctuelles (118, 118') présentant la même longueur d'onde sur le plan de pupille (120) respectif d'au moins un œil (114) d'un utilisateur,
**caractérisé en ce que**
l'au moins un faisceau lumineux (148) est généré par la source lumineuse cohérente (134) au moyen d'au moins une source lumineuse polychromatique (136) dotée d'une source laser supercontinuum (138) et d'au moins un filtre de longueur d'onde accordable (140).

10. Procédé (212) selon la revendication précédente, **caractérisé en ce que** le guidage de chaque paire de sous-faisceaux lumineux (156, 156') superposés selon l'étape c) s'effectue de telle sorte que les deux motifs d'interférence (124, 124') soient respectivement soumis à une formation d'image sur la rétine (128) respective de l'au moins un œil (114) de l'utilisateur.

11. Programme informatique pour générer au moins deux sources lumineuses ponctuelles (118, 118') de même longueur d'onde sur le plan de pupille (120) respectif d'au moins un œil (114) d'un utilisateur, comprenant des instructions qui, lors de l'exécution du programme par un ordinateur, amènent celui-ci à effectuer les étapes suivantes au moyen du dispositif (116) selon la revendication 1 :
a) générer au moins un faisceau lumineux (148) au moyen de l'au moins une source lumineuse cohérente (134) ;
b) séparer l'au moins un faisceau lumineux (148) en deux paires de sous-faisceaux lumineux (156, 156') au moyen de l'au moins un dispositif optique, superposer les sous-faisceaux lumineux (156, 156') respectifs de chaque paire et régler le contraste et la phase spatiale dans un motif d'interférence (124, 124') formé pour chaque paire par la superposition des deux sous-faisceaux lumineux (156, 156') au moyen de l'au moins un élément numérique de modulation de lumière (150), dans lequel l'élément numérique de modulation de lumière (150) est un dispositif optique qui comporte une pluralité d'éléments optiques pouvant être commandés individuellement et conçus pour moduler le faisceau lumineux entrant (148) ; et
c) guider chaque paire des sous-faisceaux lumineux (156, 156') superposés afin de générer au moins deux sources lumineuses ponctuelles (118, 118') présentant la même longueur d'onde sur le plan de pupille (120) respectif d'au moins un œil (114) d'un utilisateur,
**caractérisé en ce que**
l'au moins un faisceau lumineux (148) est généré par la source lumineuse cohérente (134) au moyen d'au moins une source lumineuse polychromatique (136) dotée d'une source laser supercontinuum (138) et d'au moins un filtre de longueur d'onde accordable (140).
